# FASCICULE DE BREVET EUROPEEN

(11) **EP 4 356 351 B1**
(45) Date de publication et mention de la délivrance du brevet: **21.05.2025**
(21) Numéro de dépôt: 22733638.5
(22) Date de dépôt: 17.06.2022
(51) Int. Cl.: A61B 3/00, A61B 3/04, G02C 7/02, G06T 1/00

(54) **PROCÉDÉ DE SIMULATION DE PRODUITS OPTIQUES**
VERFAHREN ZUR SIMULATION VON OPTISCHEN PRODUKTEN
METHOD FOR SIMULATING OPTICAL PRODUCTS

(30) Priorité: 18.06.2021 FR 2106475
(43) Date de publication de la demande: 24.04.2024
(73) Titulaire: ACEP France, 75017 Paris (FR)
(72) Inventeur: SAYAG, Jean-philippe, 75017 PARIS (FR)
(74) Mandataire: Oudin, Stéphane
(86) Numéro de dépôt international: PCT/EP2022/066604
(87) Numéro de publication internationale: WO 2022/263652

(56) Documents cités:
- EP-A1- 2 899 585
- WO-A1-2017/134275
- WO-A1-2020/243771
- FR-A1- 3 067 151
- US-A1- 2017 188 813
- US-A1- 2020 219 326
- US-B2- 8 823 742

## Description

### Domaine technique de l'invention

La présente invention concerne le domaine de l'optique et plus particulièrement le domaine de l'essayage de produits optiques de type verres ou lentilles, à l'aide de la réalité augmentée.

### Etat de la technique

Dans le domaine de l'essayage de produits optiques, il est connu d'utiliser les informations concernant la vue d'un individu contenues dans son ordonnance pour sélectionner des verres ou des lentilles de correction appropriés à sa vue et les fabriquer en vue d'un essayage. L'inconvénient de ce type d'essayage réside dans le fait que si le produit optique sélectionné n'est pas approprié au patient, il est jeté et un autre produit optique doit être fabriqué à nouveau.

Pour pallier à cet inconvénient, on connaît bien un procédé de simulation de lentilles en réalité augmentée qui à l'aide d'une tablette permet à un utilisateur de simuler l'effet d'une lentille ou d'un verre sur une image de l'environnement de l'utilisateur capturée en temps réel. Cependant, ce type de procédé ne permet pas de simuler de façon réaliste l'effet d'un verre ou d'une lentille à variation de puissance, c'est-à-dire d'un verre ou d'une lentille caractérisé(e) par le fait qu'il ou elle offre, sur toute sa surface, des puissances différentes dédiées à des distances de vision différentes. En effet, ce système de simulation ne prend pas en compte la distance des différents éléments de l'environnement avec la tablette et ne permet donc pas de restituer le plus fidèlement possible la perception en 3D du champs visuel perçu en fonction du design de la lentille représentée. Ainsi, ce type de système apporte une expérience de simulation peu représentative de la réalité pour un porteur notamment dans le cas de la simulation de verres progressifs destinés à corriger une vision dites « de prés » et une vision dites « de loin ». Le document US 8 823 742 B2 qui est considéré comme représentant l'état de la technique le plus proche divulgue un procédé standard de simulation de lentilles ou de verres optiques en réalité augmentée à l'aide d'un appareil mobile comportant un périphérique d'entrée et un périphérique d'affichage. Ledit procédé comprend une phase de capture d'une image d'un environnement d'un utilisateur à l'aide d'au moins un élément de capture d'images.

### Résumé de l'invention

Le but de la présente invention est donc de pallier les inconvénients précédemment cités et de proposer un procédé de simulation de verres ou de lentilles optiques basé sur la réalité augmentée permettant une restitution réaliste de l'effet de verres ou de lentilles optiques sur la perception visuelle d'un environnement composé d'éléments situés à différentes distances de l'utilisateur.

Conformément à l'invention, il est donc proposé un procédé de simulation de lentilles ou de verres optiques en réalité augmentée à l'aide d'un appareil mobile comportant au moins un périphérique d'entrée et un périphérique d'affichage, ledit procédé étant remarquable en ce qu'il comporte au moins,
une phase de capture (P1) comportant au moins l'étape suivante :
   a1) Capture d'au moins une image d'un environnement d'un utilisateur à l'aide d'au moins un élément de capture d'images;
une phase d'acquisition de données (P2), comportant au moins l'étape suivante :
   b1) Détermination de la distance entre l'appareil mobile et au moins un objet de l'environnement à l'aide d'au moins une unité de mesure;
et une phase de simulation (P3) mise en œuvre par ladite au moins une unité de traitement, comportant au moins l'étape suivante :
   c1) Application d'un verre ou d'une lentille virtuelle reproduisant l'effet optique d'un verre ou d'une lentille réelle de correction optique déterminée sur ladite au moins une image de l'environnement en fonction des données acquises lors de la phase d'acquisition de données (P2) et affichage de la simulation sur un périphérique d'affichage.

On appelle « appareil mobile » un appareil informatique portatif utilisable de manière autonome. Il s'agit par exemple, d'une tablette, d'un smartphone ou d'un ordinateur portable.

On appelle « utilisateur », l'individu utilisant l'appareil mobile dans le but d'obtenir une simulation de lentilles ou de verres de correction, il peut s'agir par exemple, d'un patient pour lequel un produit de correction a été conçu ou encore d'un opticien.

On appelle « lentille » une orthèse optique transparente, très fine et concave, que l'on pose sur la cornée de l'œil pour corriger les défauts de vision. On parle de « type de lentille » pour désigner des lentilles proposant différents types de corrections optiques, qui présentent différents types de revêtements ou de teintes.

On appelle « périphérique d'entrée » un équipement informatique périphérique permettant de fournir des données à un système de traitement de l'information tel qu'un ordinateur. Il s'agit par exemple d'un clavier, d'une souris, d'un écran tactile (l'écran tactile peut être considéré comme un périphérique d'entrée et de sortie à la fois). Ce périphérique peut être intégré à l'appareil mobile donc faire partie intégrante de l'appareil ou transmettre ses données à celui-ci au moyen d'une connexion avec ou sans fil.

On appelle « périphérique d'affichage » un périphérique de sortie permettant d'afficher des informations, il s'agit par exemple de l'écran de l'appareil mobile ou d'un écran qui reçoit des données de l'appareil mobile par une connexion avec ou sans fil.

On appelle « élément de capture d'image », un appareil de type appareil photo ou caméra. Cet appareil peut être intégré à l'appareil mobile (par exemple, caméra d'un smartphone ou d'une tablette) ou être indépendant et connecté à l'appareil mobile.

On appelle « unité de mesure », un instrument ou une application permettant la mesure de distance. Cet instrument peut être intégré à l'appareil mobile ou transmettre ses données à celui-ci au moyen d'une connexion avec ou sans fil.

On appelle « unité de traitement » l'ensemble des éléments de l'appareil mobile (processeur, mémoire...) servant au stockage à l'exploitation des informations sur l'environnement et la vue d'un futur porteur.

On appelle « lentille virtuelle » un filtre d'image crée en réalité augmentée et permettant de créer des effets optiques sur une image (création de zones floues par exemple, changement de la couleur de l'image etc...)

Préférentiellement, lors de l'étape c1, ledit verre ou ladite lentille virtuelle est choisie dans une base de données préexistante de verres ou de lentilles de cartographies différentes modifiables ou est complétement créée selon les besoins.

On appelle « cartographie » d'un verre ou d'une lentille, la manière dont sont agencées les différentes zones de puissance de correction optique sur sa surface.

Avantageusement, pendant la phase de simulation (P3), l'utilisateur peut choisir de simuler un verre ou une lentille: multifocale, à variation de puissance, progressif, dégressif, à positionnement spécifique, de suppression, de progression de la myopie, à teinte fixe ou dégradée ou bidégradée, photochromique, polarisant, de revêtement, ou anti-fatigue.

De manière préférentielle, la phase d'acquisition de données (P2) comporte une étape b2 qui se déroule lors de l'observation de l'environnement par un porteur à travers le périphérique d'affichage de l'appareil mobile et qui consiste à déterminer les mouvements de la tête du porteur, et la distance entre l'utilisateur et l'appareil mobile à l'aide d'un capteur de mouvement.

On appelle « porteur », un patient pour lequel sont destinés les lentilles ou les verres.

De manière encore plus préférentielle, la détermination des mouvements au niveau de la tête du porteur consiste à détecter les mouvements du visage et des yeux du porteur par rapport à l'appareil mobile à l'aide d'un capteur de mouvement.

De manière avantageuse, l'étape a1 est une étape de capture en temps réel d'au moins une image d'un environnement d'un utilisateur à l'aide d'au moins un élément de capture d'images.

Préférentiellement, à l'étape b1, l'unité de mesure permettant de déterminer la distance entre l'appareil mobile et l'objet est un dispositif ou une application télémétrique.

On appelle « dispositif ou application télémétrique », un dispositif ou une application permettant de mesurer la distance d'un objet par des moyens informatiques (applications basées sur le calcul de distance suite à la capture d'une image de l'objet par exemple), optique (télémétrie laser par exemple), acoustique (sonar par exemple) ou radioélectrique (radar par exemple).

De manière avantageuse, l'unité de mesure est un télémètre à mesure laser, à ultra-sons, acoustiques ou une application de mesure à distance.

De manière préférée, au moins l'élément de capture d'images, et l'unité de mesure sont intégrés à l'appareil mobile.

Avantageusement, l'appareil mobile est une tablette ou un smartphone et le périphérique d'affichage est l'écran de ladite tablette ou dudit smartphone.

Préférentiellement, à l'étape c1, la simulation est affichée sur le périphérique d'affichage de l'appareil mobile.

### Brève description des figures

D'autres avantages et caractéristiques ressortiront mieux de la description qui va suivre d'un mode d'exécution d'un procédé selon l'invention, en référence aux figures annexées sur lesquelles :
[Fig.1] est un organigramme des étapes du procédé conforme à l'invention,
[Fig.2] est une vue schématique d'un mode de réalisation d'un système pour mettre en œuvre le procédé conforme à l'invention.

### Description des modes de réalisation

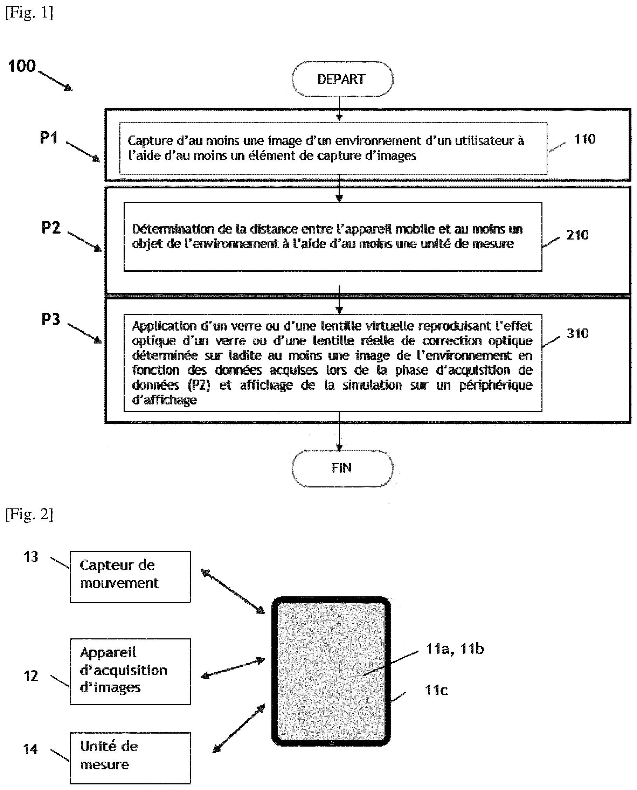

Conformément aux figures 1 et 2, il est donc décrit un procédé 100 de simulation de lentilles ou de verres optiques en réalité augmentée à l'aide d'un appareil mobile 11 comportant au moins un périphérique d'entrée 11a et un périphérique d'affichage 11b, ledit procédé 100 étant remarquable en ce qu'il comporte au moins, une phase de capture (P1) comportant au moins l'étape suivante :
a1) Capture d'au moins une image d'un environnement d'un utilisateur à l'aide d'au moins un élément de capture d'images 12, 110 ;
une phase d'acquisition de données (P2), comportant au moins l'étape suivante :
b1) Détermination de la distance entre l'appareil mobile 11 et au moins un objet de l'environnement à l'aide d'au moins une unité de mesure 14, 210;
et une phase de simulation (P3) mise en œuvre par ladite au moins une unité de traitement 11c de l'appareil mobile, comportant au moins l'étape suivante :
c1) Application d'un verre ou d'une lentille virtuelle reproduisant l'effet optique d'un verre ou d'une lentille réelle de correction optique déterminée, sur ladite au moins une image de l'environnement, en fonction des données acquises lors de la phase d'acquisition de données (P2) et affichage de la simulation sur un périphérique d'affichage, 310;

L'appareil mobile 11 pourra être une tablette, un smartphone ou un ordinateur portable. Quel que soit l'appareil mobile 11, le périphérique d'affichage 11b sera l'écran de l'appareil mobile 11 ou un écran indépendant connecté à l'appareil mobile 11 par une connexion avec ou sans fil (par exemple, connexion par câble USB, connexion Bluetooth...). Si l'appareil mobile 11 est une tablette ou un smartphone, le périphérique d'entrée 11a sera préférentiellement un écran tactile. Si l'appareil mobile 11 est un PC portable, le périphérique d'entrée 11a sera un clavier et/ou une souris et/ ou un écran tactile. Ce périphérique d'entrée 11a aura pour but de permettre de sélectionner les différents types de verres ou de lentilles à simuler ou de créer des verres ou des lentilles avec de nouvelles cartographies. Il pourra également servir à saisir la correction optique du produit pour la simulation.

L'élément de capture d'images 12 est un appareil de type appareil photo ou caméra qui peut être intégré à l'appareil mobile (appareil photo d'une tablette ou d'un smartphone par exemple) ou connecté à celui-ci par une connexion avec ou sans fil (par exemple, connexion par câble USB, connexion Bluetooth...). Cet élément de capture d'images 12 a pour but de capturer une image de l'environnement et de transmettre cette image au périphérique d'affichage 11b de l'appareil mobile 11. L'image capturée pourra être une image prise en temps réel ou en différé.

L'unité de traitement 11c crée ensuite une image de verre ou de lentille sur mesure en traitant l'ensemble des données acquises lors de la phase d'acquisition P2 des données et en prenant en compte un certain nombre d'informations relatives à la fabrication générale de verres ou de lentilles optiques préalablement stockées sur l'appareil mobile 11. La prise en compte de la distance environnement -appareil mobile 11, est un facteur déterminant dans la précision de la simulation de la lentille ou du verre. En effet, cela permet de restituer le plus fidèlement possible ce qui peut être perçu par un œil amétrope ou emmétrope équipé d'un dispositif de compensation de son amétropie et/ou de sa presbytie. Toute lentille ou verre à variation de puissance offre, sur toute sa surface, une cartographie de puissances différentes dédiées à des distances de vision pouvant être comprises entre l'infini et 30 cm. Cela implique que dans le champ visuel de l'utilisateur, tout objet perçu à travers la zone optique qui ne correspond pas à sa position d'éloignement, sera perçu flou. L'utilisateur est donc obligé de modifier sa direction de regard afin d'utiliser la zone optique qui correspond à l'éloignement de l'objet visé. Par exemple, dans le cas d'une lentille progressive, si le sujet baisse les yeux pour descendre un escalier, il utilise la zone optique réservée à la vision de près et les marches visées seront vues floues. Autre exemple : Si un sujet regarde au loin avec une lentille progressive, les objets situés en périphérie de son champ visuel horizontal seront perçus flous du fait des iso-astigmatismes présents latéralement sur la surface de ladite lentille progressive.

L'avantage de cette mesure de distance (étape b1), est donc de restituer le plus fidèlement possible la perception en 3D du champ visuel perçu en fonction de la cartographie de la lentille ou du verre sélectionné et ainsi démontrer les différences existantes entre différents types de lentilles ou de verres.

Cette lentille ou ce verre élaboré en réalité augmentée est superposé à l'image de l'environnement capturé par l'élément de capture d'images 12.

L'utilisateur qui regarde le périphérique d'affichage voit alors exactement ce qu'il verrait s'il portait de véritables lentilles ou verres de correction. Suite à cette simulation si le produit oculaire convient, la fabrication du produit peut être lancée.

Préférentiellement, lors de l'étape c1, ledit verre ou ladite lentille virtuelle est choisie dans une base de données préexistante de verres ou de lentilles de cartographies différentes modifiables ou est complétement créée selon les besoins. L'utilisateur peut sélectionner dans une base de données un verre ou une lentille avec une cartographie « type », l'unité de traitement 11c générera alors une lentille virtuelle de correction optique particulière selon cette cartographie : par exemple, lentille progressive. L'utilisateur pourra au besoin modifier la cartographie d'une lentille ou d'un verre de la base de données ou choisir de créer un nouveau verre ou une nouvelle lentille virtuelle avec une cartographie sur-mesure de manière à répondre aux besoins du futur porteur.

Avantageusement, pendant la phase de simulation (P3), l'utilisateur peut choisir de simuler un verre ou une lentille: multifocale, à variation de puissance, progressif, dégressif, à positionnement spécifique, de suppression, de progression de la myopie, à teinte fixe ou dégradée ou bidégradée, photochromique, polarisant, de revêtement, ou anti-fatigue.

De manière préférentielle, la phase d'acquisition de données (P2) comporte une étape b2 qui se déroule lors de l'observation de l'environnement par un porteur à travers le périphérique d'affichage 11b de l'appareil mobile 11 et qui consiste à déterminer les mouvements de la tête du porteur, et la distance entre le porteur et l'appareil mobile 11 à l'aide d'un capteur de mouvement 13.

Pendant la phase d'acquisition de données (P2), le comportement visuel du porteur par rapport à l'image de l'environnement qu'il observe sur le périphérique d'affichage 11b est déterminé et enregistré, les distances entre lui et l'appareil mobile 11 et entre l'appareil mobile 11 et un objet de l'environnement sont mesurées. Ces données vont être transmises à l'unité de traitement 11c qui va les enregistrer et les interpréter afin de déterminer la correction optique nécessaire au porteur et afin de simuler une lentille ou un verre correspondant à cette correction.

De manière encore plus préférentielle, la détermination des mouvements au niveau de la tête du porteur consiste à détecter les mouvements du visage et des yeux du porteur par rapport à l'appareil mobile 11 à l'aide d'un capteur de mouvement 13. Le capteur de mouvement 13 peut prendre la forme de n'importe quel type de capteur qui permet de détecter le mouvement. De manière préférée, il s'agira d'un capteur optique qui pourra faire partie de l'appareil mobile 11 ou être connecté à celui-ci par une connexion avec ou sans fil. Le capteur 13 permet de détecter les mouvements d'un porteur qui observe l'environnement en tenant dans ses mains l'appareil mobile 11. Il peut détecter par exemple que le porteur approche ou recule l'appareil mobile 11, que le porteur bouge l'appareil 11 vers le haut, le bas, à gauche ou à droite ou encore déterminer la distance entre le porteur et l'appareil mobile 11 à l'aide d'un capteur d'accélération.

De manière avantageuse, l'étape a1 est une étape de capture en temps réel d'au moins une image d'un environnement d'un utilisateur à l'aide d'au moins un élément de capture d'images 12.

Préférentiellement, à l'étape b1, l'unité de mesure 14 permettant de déterminer la distance entre l'appareil mobile 11 et l'objet est un dispositif ou une application télémétrique. On appelle « dispositif ou application télémétrique », un dispositif ou une application permettant de mesurer la distance d'un objet par des moyens informatiques (applications basées sur le calcul de distance suite à la capture d'une image de l'objet par exemple), optique (télémétrie laser par exemple), acoustique (sonar par exemple) ou radioélectrique (radar par exemple).

Si l'appareil mobile 11 est une tablette ou un smartphone, l'unité de mesure 14 pourra être une application de mesure à distance comme par exemple « Mapulator » ou « EasyMeasure » sur Android et iOS. « Easy mesure » permet de mesurer à l'aide d'un appareil de capture d'image 12 en temps réel et en réalité augmentée de manière précise plusieurs distances dont la distance entre l'appareil mobile 11 et un objet. Dans un mode de réalisation particulier, l'unité de mesure 14 sera un télémètre laser connecté à l'appareil mobile 11 par une connexion avec ou sans fil et dont le fonctionnement sera le suivant : Il projettera un rayon laser sur un objet de l'environnement qui renverra à son tour le rayon lumineux, le télémètre calculera alors le déphasage entre l'émission et la réception.

De manière préférentielle, l'appareil mobile 11 doté de la technologie LiDAR (Light Detection and Ranging). La technologie LiDAR permet une détection et une estimation de la distance par la lumière ou par laser. Cette technologie de mesure à distance est possible grâce à l'analyse des propriétés d'un faisceau de lumière renvoyé vers son émetteur (appareil mobile 11). Le LiDAR utilise la lumière pour mesurer la distance à l'aide de lasers à spectre vert pulsé invisibles. Ces impulsions (qui se produisent des milliers de fois par minute) mesurent le temps nécessaire à la lumière pour revenir au capteur. Ce faisant, il dresse une "image" de l'environnement devant le scanner.

De manière avantageuse, l'unité de mesure 14 est un télémètre à mesure laser, à ultra-sons, acoustiques ou une application de mesure à distance.

De manière préférée, l'élément de capture d'images 12, et l'unité de mesure 14 sont intégrés à l'appareil mobile 11. Le terme « intégré » signifie ici que l'appareil mobile 11 a le contrôle de ces différents éléments, soit ces éléments font partie intégrante du corps/boîtier l'appareil mobile 11, soit ils sont externes à celui -ci (ils ne font pas partie du boîtier de l'appareil mobile 11) mais ne peuvent fonctionner que sous le contrôle de l'appareil mobile 11. De cette manière, la simulation est plus facile à mettre en place. Le capteur de mouvement 13 peut lui aussi être intégré à l'appareil mobile 11. Bien évidemment, si des capteurs de mouvements 13, des éléments de captures d'images 12 et/ou des unités de mesure 14 différentes de celles intégrés à l'appareil mobile 11 sont nécessaires, il est tout à fait possible d'utiliser le ou les appareils appropriés en les connectant par une connexion avec ou sans fil à l'appareil mobile 11.

Avantageusement, l'appareil mobile 11 est une tablette ou un smartphone et le périphérique d'affichage 11b est l'écran de ladite tablette ou dudit smartphone. On choisira parmi les appareils mobiles 11 existants un appareil assez puissant pour supporter ce type de simulation. On choisira par exemple une tablette de type iPad. Sur la tablette ou le smartphone l'élément de capture d'images 12 et l'unité de mesure 14 seront préférentiellement à l'arrière de l'appareil 11, alors que le capteur d'image sera plutôt à l'avant du côté de l'écran.

Encore plus avantageusement, la simulation est affichée sur le périphérique d'affichage 11b de l'appareil mobile 11.

Enfin, il va de soi que les exemples de procédés 100 conformes à l'invention qui viennent d'être décrits ne sont que des illustrations particulières, en aucun cas limitatives de l'invention.

## Revendications

1. Procédé (100) de simulation de lentilles ou de verres optiques en réalité augmentée à l'aide d'un appareil mobile (11) comportant au moins un périphérique d'entrée (11a) et un périphérique d'affichage (11b), ledit procédé (100) comportant au moins, une phase de capture (P1) comportant au moins l'étape suivante :
a1) Capture d'au moins une image d'un environnement d'un utilisateur à l'aide d'au moins un élément de capture d'images (12) (110);
une phase d'acquisition de données (P2); étant **caractérisé en ce qu'**il comporte au moins l'étape suivante :
b1) Détermination de la distance entre l'appareil mobile (11) et au moins un objet de l'environnement à l'aide d'au moins une unité de mesure (14) (210);
et une phase de simulation (P3) mise en œuvre par au moins une unité de traitement (11c) de l'appareil mobile (11), comportant au moins l'étape suivante :
c1) Application d'un verre ou d'une lentille virtuelle reproduisant l'effet optique d'un verre ou d'une lentille réelle de correction optique déterminée, sur ladite au moins une image de l'environnement, en fonction des données acquises lors de la phase d'acquisition de données (P2) et affichage de la simulation sur un périphérique d'affichage (310);

2. Procédé (100) selon la revendication 1 **caractérisé en ce que** lors de l'étape c1, ledit verre ou ladite lentille virtuelle est choisie dans une base de données préexistante de verres ou de lentilles de cartographies différentes modifiables ou est créée avec une cartographie sur-mesure.

3. Procédé (100) selon l'une quelconques des revendications précédentes en ce que pendant la phase de simulation (P3), l'utilisateur choisit de simuler un verre ou une lentille : multifocale, à variation de puissance, progressif, dégressif, à positionnement spécifique, de suppression, de progression de la myopie, à teinte fixe ou dégradée ou bidégradée, photochromique, polarisant, de revêtement, ou anti-fatigue.

4. Procédé (100) selon l'une quelconque des revendications précédentes **caractérisé en ce que** la phase d'acquisition de données (P2) comporte une étape b2 qui se déroule lors de l'observation de l'environnement par un porteur à travers le périphérique d'affichage (11b) de l'appareil mobile et qui consiste à déterminer les mouvements de la tête du porteur, et la distance entre le porteur et l'appareil mobile (11) à l'aide d'un capteur de mouvement (13).

5. Procédé (100) selon la revendication 4 **caractérisé en ce que**, la détermination des mouvements au niveau de la tête du porteur consiste à détecter les mouvements du visage et des yeux du porteur par rapport à l'appareil mobile (11) à l'aide d'un capteur de mouvement (13).

6. Procédé (100) selon l'une quelconque des revendications précédentes **caractérisé en ce que** l'étape a1 est une étape de capture en temps réel d'au moins une image d'un environnement d'un utilisateur à l'aide d'au moins un élément de capture d'images (12).

7. Procédé (100) selon l'une quelconque des revendications précédentes **caractérisé en ce que**, à l'étape b1, l'unité de mesure (14) permettant de déterminer la distance entre l'appareil mobile (11) et l'objet est un dispositif ou une application télémétrique.

8. Procédé (100) selon la revendication 7, **caractérisé en ce que** l'unité de mesure (14) est un télémètre à mesure laser, à ultra-sons, acoustiques ou une application de mesure à distance.

9. Procédé (100) selon l'une quelconques des revendications précédentes **caractérisé en ce qu'**au moins l'élément de capture d'images (12), et l'unité de mesure (14) sont intégrés à l'appareil mobile (11).

10. Procédé (100) selon l'une quelconques des revendications précédentes **caractérisé en ce que** l'appareil mobile (11) est une tablette ou un smartphone et **en ce que** le périphérique d'affichage (11b) est l'écran de ladite tablette ou dudit smartphone.

## Patentansprüche

1. Verfahren (100) zur Simulation von optischen Linsen oder Gläsern in der Erweiterten Realität mithilfe eines mobilen Geräts (11), das mindestens ein Eingabegerät (11a) und ein Anzeigegerät (11b) umfasst, wobei das Verfahren (100) mindestens
eine Aufnahmephase (P1), die mindestens den folgenden Schritt umfasst:
a1) Aufnehmen mindestens eines Bildes einer Umgebung eines Benutzers mithilfe mindestens eines Bildaufnahmeelements (12) (110);
eine Datenerfassungsphase (P2); **dadurch gekennzeichnet, dass** sie mindestens den folgenden Schritt umfasst:
b1) Bestimmen des Abstands zwischen dem mobilen Gerät (11) und mindestens einem Objekt in der Umgebung mithilfe von mindestens einer Messeinheit (14) (210);
und eine Simulationsphase (P3) umfasst, die von mindestens einer Verarbeitungseinheit (11c) des mobilen Geräts (11) implementiert wird und mindestens den folgenden Schritt umfasst:
c1) Anwenden eines virtuellen Glases oder einer virtuellen Linse, die den optischen Effekt eines realen Glases oder einer realen Linse mit bestimmter optischer Korrektur reproduziert, auf das mindestens eine Bild der Umgebung in Abhängigkeit von den während der Datenerfassungsphase (P2) erfassten Daten und Anzeigen der Simulation auf einem Anzeigegerät (310;

2. Verfahren (100) nach Anspruch 1, **dadurch gekennzeichnet, dass** in Schritt c1 das virtuelle Glas oder die virtuelle Linse aus einer vorbestehenden Datenbank von Gläsern oder Linsen mit verschiedenen veränderbaren Abbildungen ausgewählt oder mit einer maßgeschneiderten Abbildung erstellt wird.

3. Verfahren (100) nach einem der vorhergehenden Ansprüche, in dem der Benutzer während der Simulationsphase (P3) wählt, ein Glas oder eine Linse zu simulieren: multifokal, mit variabeler Brechkraft, progressiv, degressiv, mit spezifischer Positionierung zur Unterdrückung der Myopieprogression, mit fester oder abgestufter oder zweifarbig abgestufter Tönung, phototochrom, polarisierend, mit Beschichtung oder mit Anti-Ermüdungseffekt.

4. Verfahren (100) nach einem der vorhergehenden Ansprüche, **dadurch gekennzeichnet, dass** die Datenerfassungsphase (P2) einen Schritt b2 umfasst, der während der Beobachtung der Umgebung durch einen Träger über das Anzeigegerät (11b) des mobilen Geräts abläuft und darin besteht, die Bewegungen des Kopfes des Trägers und den Abstand zwischen dem Träger und dem mobilen Gerät (11) mithilfe eines Bewegungssensors (13) zu bestimmen.

5. Verfahren (100) nach Anspruch 4, **dadurch gekennzeichnet, dass** das Bestimmen der Bewegungen am Kopf des Trägers darin besteht, die Bewegungen des Gesichts und der Augen des Trägers in Bezug auf das mobile Gerät (11) mithilfe eines Bewegungssensors (13) zu detektieren.

6. Verfahren (100) nach einem der vorhergehenden Ansprüche, **dadurch gekennzeichnet, dass** der Schritt a1 ein Schritt des Aufnehmens von mindestens einem Bild einer Umgebung eines Benutzers mithilfe von mindestens eines Bildaufnahmeelemente (12) in Echtzeit ist.

7. Verfahren (100) nach einem der vorhergehenden Ansprüche, **dadurch gekennzeichnet, dass** in Schritt b1 die Messeinheit (14), die es ermöglicht, den Abstand zwischen dem mobilen Gerät (11) und dem Objekt zu bestimmen, eine telemetrische Vorrichtung oder Anwendung ist.

8. Verfahren (100) nach Anspruch 7, **dadurch gekennzeichnet, dass** die Messeinheit (14) ein Entfernungsmesser mit Laser-, Ultraschall- oder akustischer Messung oder eine Fernmessanwendung ist.

9. Verfahren (100) nach einem der vorhergehenden Ansprüche, **dadurch gekennzeichnet, dass** mindestens das Bildaufnahmeelement (12) und die Messeinheit (14) in das mobile Gerät (11) integriert sind.

10. Verfahren (100) nach einem der vorhergehenden Ansprüche, **dadurch gekennzeichnet, dass** das mobile Gerät (11) ein Tablet oder Smartphone ist und dass das Anzeigegerät (11b) der Bildschirm des Tablets oder Smartphones ist.

## Claims

1. A method (100) for simulating optical lenses in augmented reality using a mobile apparatus (11) including at least one input peripheral (11a) and one display peripheral (11b), said method (100) including at least,
one capture phase (P1) including at least the following step:
a1) Capturing at least one image of a user's environment using at least one image capture element (12) (110);
one data acquisition phase (P2); being **characterised in that** it includes at least the following step:
b1) Determining the distance between the mobile apparatus (11) and at least one object in the environment using at least one measurement unit (14) (210);
and one simulation phase (P3) implemented by at least one processing unit (11c) of the mobile apparatus (11), including at least the following step:
c1) Applying a virtual lens reproducing the optical effect of an actual determined optical-correction lens, on said at least one image of the environment, depending on the data acquired during the data acquisition phase (P2) and displaying the simulation on a display peripheral (310);

2. The method (100) according to claim 1, **characterised in that** during step c1, said virtual lens is selected from a pre-existing database of lenses with different modifiable mappings or is created with a custom mapping.

3. The method (100) according to any one of the preceding claims, **characterised in that** during the simulation phase (P3), the user chooses to simulate a lens: multifocal, with power variation, progressive, digressive, with specific positioning, for suppression, of myopia progression, with fixed or gradient or bi-gradient tint, photochromic, polarising, coating, or anti-fatigue.

4. The method (100) according to any one of the preceding claims, **characterised in that** the data acquisition phase (P2) includes a step b2 which takes place during the observation of the environment by a wearer through the display peripheral (11b) of the mobile apparatus and which consists in determining the movements of the wearer's head, and the distance between the wearer and the mobile apparatus (11) using a movement sensor (13).

5. The method (100) according to claim 4, **characterised in that**, the determination of the movements at the wearer's head consists in detecting the movements of the wearer's face and eyes relative to the mobile apparatus (11) using a movement sensor (13).

6. The method (100) according to any one of the preceding claims, **characterised in that** step a1 is a step of capturing in real time at least one image of a user's environment using at least one image capture element (12).

7. The method (100) according to any one of the preceding claims, **characterised in that**, in step b1, the measurement unit (14) allowing determining the distance between the mobile apparatus (11) and the object is a telemetric device or application.

8. The method (100) according to claim 7, **characterised in that** the measurement unit (14) is a laser, ultrasonic, acoustic measuring rangefinder or a remote measuring application.

9. The method (100) according to any one of the preceding claims, **characterised in that** at least the image capture element (12), and the measurement unit (14) are integrated into the mobile apparatus (11).

10. The method (100) according to any one of the preceding claims, **characterised in that** the mobile apparatus (11) is a tablet or a smartphone and **in that** the display peripheral (11b) is the screen of said tablet or of said smartphone.
